# EUROPEAN PATENT SPECIFICATION

(11) **EP 3 167 082 B1**
(45) Date of publication and mention of the grant of the patent: **18.04.2018**
(21) Application number: 14777868.2
(22) Date of filing: 10.07.2014
(51) Int. Cl.: C14B 17/04, C14B 17/14, G01N 33/44, C14B 5/00, C14B 17/00

(54) **DEVICE FOR DETECTION, MARKING AND CUTTING OF LEATHER**
VORRICHTUNG ZUR ERKENNUNG, MARKIERUNG UND ZUM SCHNEIDEN VON LEDER
DISPOSITIF POUR LA DÉTECTION, LE MARQUAGE ET LA COUPE DU CUIR

(43) Date of publication of application: 17.05.2017
(73) Proprietor: Camoga S.p.A., 20032 Cormano (Milano) (IT)
(72) Inventor: MASCETTI, Matteo, 20032 Cormano (Milano) (IT)
(74) Representative: Tarabbia, Luigi
(86) International application number: PCT/IT2014/000184
(87) International publication number: WO 2016/006007

(56) References cited:
- WO-A1-2011/030360
- DE-A1- 1 903 867
- DE-A1- 19 737 703
- GB-A- 2 188 170

## Description

The object of the present invention is an apparatus for detecting defects in at least one portion of leather, and for cutting the portion, which enables the operations of dilation, moving and cutting said at least one portion of leather to be performed faster and more efficiently than with currently known systems.

Known apparatuses are disclosed in GB 2188170 A, DE 19737703 A1, WO 2011/030360 A1 and DE 1903867 A1.

The leather-cutting step is normally preceded by the operations of visual analysis and marking thereof, which are fundamental for appropriate execution of this subsequent cutting step. This visual analysis in practice aims to map the positioning and gravity of various types of defects such as, for example, scratches, permanent creases or grooves and the shape and extent of the contours of the specific portion of leather that will have to be cut.

The leather viewing and marking operations can be performed manually or by digital scanning, whereas the accessory operations, like moving and dilating in extent the leather in order to highlight clearly the defects, are performed currently on flat tables or on stands with extendible parts (extendible saddles). This requires the work of several people to move the portion of leather, which is normally very heavy, and to dilate it by deforming the extent thereof along its middle surface, which obviously requires significant time to perform such operations and thus entails high labour costs.

Also the cutting operations, which can be performed manually or by automatic devices, currently give rise to problems linked to the resistance of the leather to the cutting member, and to the need to keep the part subjected to cutting stationary with respect to the resting surface thereof.

The object of the present invention is to devise an apparatus for detecting defects in the leather for subsequent cutting thereof that makes the operations that are accessory to viewing and marking the leather faster than currently known systems.

Another object of the present invention is to devise an apparatus for detecting defects in leather and for the subsequent cutting of the leather, that enables cutting operations to be performed more efficiently than with currently known systems.

These objects are achieved by an apparatus according to claim 1. The curvature of the lying surface means that the leather is extended along a surface that is closer to the natural arched section of the animal from which the leather comes. In this manner, a greater opening of the fibres to the cutting blade is obtained and the formation of creases during stretching is hindered.

Further, this curvature maintains the leather more stationary with respect to the rotatable structure, also facilitating the subsequent cutting operations, as will be clear further on. A first part of the securing means and a second part of the securing means are situated respectively on two opposite edges of the lying surface. The securing means comprises one or more grippers, arranged preferably on the lying surface and configured for grasping and retaining end strips of said at least one portion of leather.

Preferably, this first part of the securing means comprises a first gripper and this second part of the securing means comprises a second gripper; in keeping with the scope of the invention there can be several grippers both for radial and orbital tensioning with respect to the lying surface. The apparatus comprises one or more cutting members and correlated movement means that are suitable for moving said cutting members on the lying surface, along at least one first direction parallel to the rotation axis of the rotatable structure and a second direction that is transverse to this rotation axis. Alternatively, the cutting movement can be achieved by the combination of the movement parallel to the rotation axis of the rotatable structure and the movement of the rotatable structure.

The cutting movement can also consist of the combination of the above three movements: two rotation and one translation movements. Preferably, the lying surface comprises at least part of a cylindrical surface.

Preferably, the lying surface is defined by a cylindrical surface. The apparatus comprises visual detection means of said at least one portion of leather, positioned in such a manner as to face the lying surface during rotation of the rotatable structure around the rotation axis.

Preferably, the apparatus comprises a frame that is suitable for carrying the visual detection means. The rotatable structure comprises at least one sector defining a part of the lying surface, and the expansion means is active on said at least one sector to translate it radially, or also rotationally translate it (depending on the needs of the moment) with respect to the rotation axis of the rotatable structure, so as to increase or decrease the extent of the lying surface.

The possibility of extending the extent of the lying surface by moving at least a part thereof radially with respect to the rotation axis combines advantageously with the curved shape of the surface, determining, also owing to the securing means, good deformation of the leather on the lying surface.

Preferably, the rotatable structure comprises a plurality of sectors defining respective adjacent parts of the lying surface, and the expansion means is active on said sectors to translate the sectors radially or rotationally translate them with respect to the rotation axis of the rotatable structure, so as to increase or decrease the extent of the lying surface.

The division into several sectors of the rotatable structure increases the deformation of the leather induced by the extension of the lying surface, and thus the visibility of the defects and the tendency of the leather to remain stationary with respect to the rotatable structure.

Preferably, the movement means of the cutting member comprises at least one slidable carriage (with relative motor drive) along at least said first direction parallel to the rotation axis of the rotatable structure by means of a main guide.

Preferably, the movement means of one or more cutting members comprises, for each member, one curved guide which is integral with the carriage.

Preferably, the curved guide extends transversely to said first direction and around the lying surface.

Preferably, the curved guide is annular.

Preferably, the curved guide follows at least one external profile of the lying surface so as to be suitable for maintaining in contact the cutting member with said at least one external profile, during the translation of said cutting member along said curved guide by a suitable motor drive.

The curvature of the lying surface, owing to which the leather remains more stationary with respect to the rotatable structure also whilst the cutting member operates, enables movement means of smaller dimensions to be used or correspondingly enables greater acceleration of such movement means to be obtained along each of the first and second directions.

Preferably, the rotation means comprises a rotating support that is suitable for dragging the rotatable structure during rotation thereof around the rotation axis, and a motor that is suitable for rotating said rotating support. Preferably, the expansion means is operationally interposed between the rotation means and the rotatable structure.

Preferably, the expansion means is operationally interposed between the rotating support and the rotatable structure.

Preferably, the lying surface is connected fluid-dynamically to sucking means that is suitable for creating a vacuum on at least part of said lying surface.

The curved shape of the lying surface, which is preferably at least partially cylindrical, enables a saving in the sucking power that is necessary for maintaining the leather sufficiently stationary with respect to the rotatable structure.

Preferably, the sucking means is at least partially integrated into the rotatable structure.

The features of the present invention will be clarified by the following description offered by way of non-limiting example of the more general concepts claimed.

The following detailed description refers to the attached tables, in which:
- figure 1 is an overall frontal view of an apparatus according to the preferred embodiment of the present invention;
- figure 2 is a side view of the embodiment in figure 1;
- figure 3 is a view of a rotatable structure of the apparatus in figure 1.

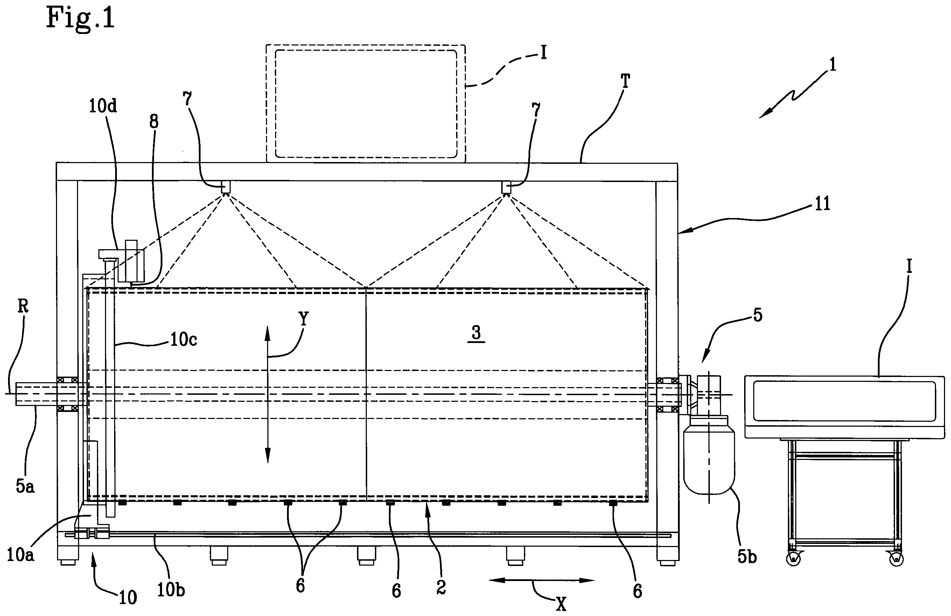

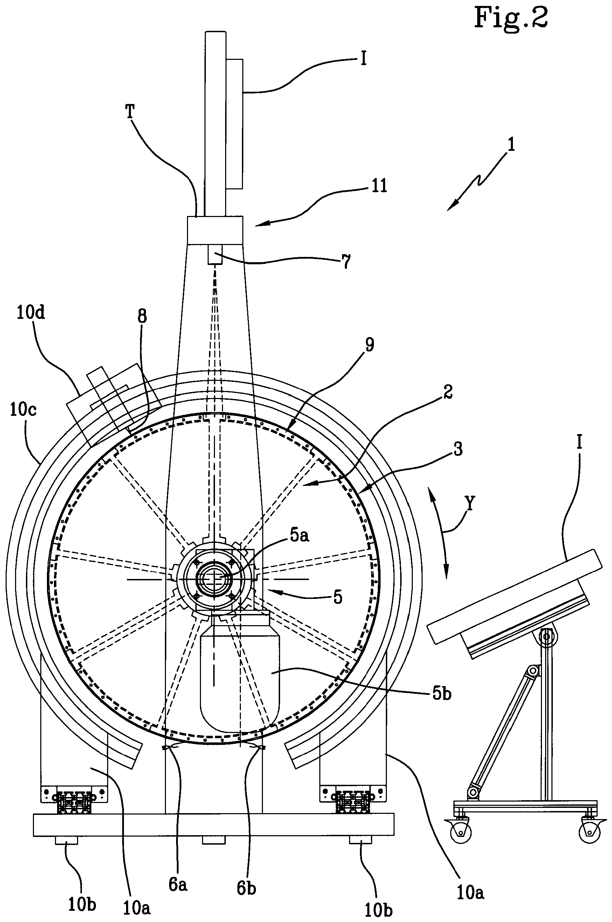

Figures 1 and 2 show an apparatus 1 for the operations of analysing and cutting at least one portion of leather, comprising a rotatable structure 2 that defines at least one external lying surface 3, of convex shape, suitable for acting as a rest and supporting this portion of leather, practically at an external contour of this rotatable structure 2.

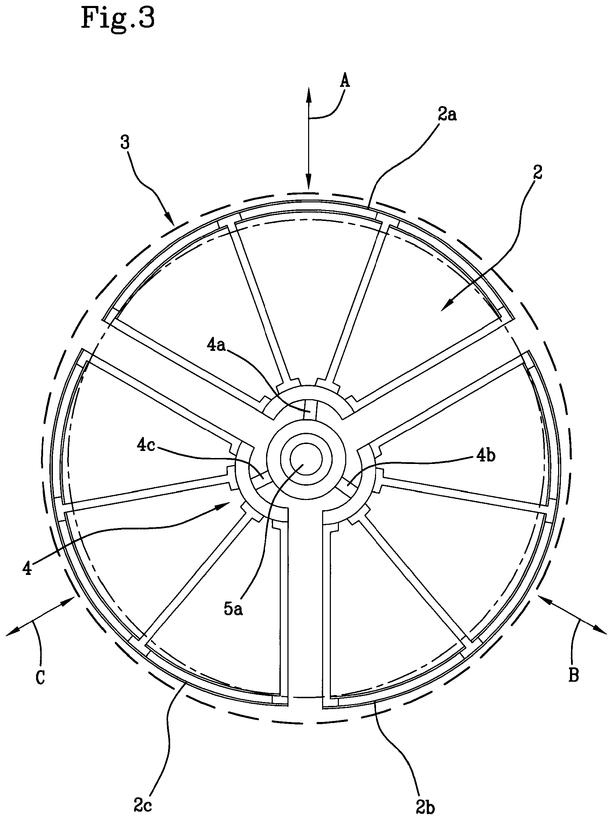

In figure 3, with respect to which the rotation axis is perpendicular, the lying surface 3 is indicated with a dashed edge around the rotatable structure 2, slightly spaced away from the structure 2 for the sake of clarity. By "lying surface" a surface is meant, possibly even only a part of which comprises solid material points, and any way on which said at least one portion of leather can be positioned.

In order to ensure that the portion of leather remains stationary with respect to the structure 2, above all to ensure appropriate execution of the cutting operations and a correct correlation between the latter and the preceding step of analysis-mapping of the defects of the leather, the apparatus 1 comprises securing means 6 between the portion of leather and the structure 2.

In the embodiment shown, as is noted in the attached figures, a first part 6a of the securing means and a second part 6b thereof are situated respectively on opposite sides of the lying surface 3, so that the expansion of the lying surface 3 causes deformation of the leather along more or less the entire extent thereof. It can in fact be affirmed that along the contour of the rotatable structure 2, at least one portion of the lying surface 3 is interposed between the parts 6a and 6b of the securing means, and that at least one part of the lying surface 3 can itself be considered to be a lying surface 3.

It can thus be said that a first part 6a of the securing means is situated on an edge opposite the edge on which a second part 6b of the securing means is situated, in the embodiment shown.

Such securing means preferably comprises one or more grippers 6a and 6b, arranged preferably on the lying surface 3 and configured for grasping and retaining end strips of said at least one portion of leather.

The structure 2 is rotatable and suitable for being rotated around a horizontal rotation axis "R" by rotation means 5, to permit the operator or the possible visual detection means 7 to view the surface of the leather, once the leather is placed around the structure 2.

In this manner the presence of several operators is not necessary for moving the leather manually, which could also be very heavy.

In order to maintain the portion of leather completely stationary during the cutting operations, the lying surface 3 is connected fluid-dynamically to sucking means 9 that is suitable for creating a vacuum on at least part of said lying surface 3. This sucking means 9, to simplify the configuration of the apparatus 1, is at least partially integrated into the rotatable structure 2. This could be achieved by a uniform distribution of a great number of sucking holes or by means of one or more localised sucking openings, which preferably have a prevalent extending dimension parallel to the rotation axis "R".

In order to permit automatic scanning of the leather, the apparatus 1 comprises visual detection means 7 of the portion of leather resting around the rotatable structure 2. This visual detection means is advantageously connected to hardware on which the software is installed that manages scanning, and to electronic interface means "I" that enables the operator to control the software.

The visual detection means 7 is positioned in such a manner as to face the lying surface 3 during rotation of the rotatable structure 2 around the rotation axis "R". In this manner almost all the surface of the portion of leather supported by the structure 2 can be mapped and controlled.

Such detection means 7 is advantageously fixed to a frame 11 that is suitable for carrying said detection means 7, and can be fixed for example to an upright or crosspiece "T", as in the embodiment shown.

Advantageously, the lying surface 3 is curved, which enables space to be saved for the same surface of leather to be analysed, and further enables the weight of the leather to be exploited (and in particular the side areas) to achieve tensioning of the leather. In other words, the curved conformation of the lying surface 3 enables the portion of leather to be "hung" on the rotatable structure 2, with clear benefits in terms of operation and efficiency of the apparatus.

In particular, it is preferable for the lying surface 3 to comprise at least part of a cylindrical surface, to simplify the geometry thereof and optimise the effects of deformation of the leather that can be obtained owing to the expansion means 4, indicated in figure 3.

In the embodiment shown the lying surface comprises a cylindrical surface, in particular the external cylindrical surface of a cylindrical roller defining the aforesaid rotatable structure 2.

In order to perform these cutting operations, the apparatus comprises a cutting member 8 and movement means 10 suitable for moving the apparatus on said lying surface 3. The cutting member 8 can be moved along at least one first direction parallel to the rotation axis "R" of the structure 2 and possibly along a second direction transverse to this rotation axis "R".

In the embodiment shown, the movement of the cutting member 8 along the first direction occurs according to the arrow "X" in figure 1, thus horizontally, whereas the movement along the second direction is orbital around the rotation axis "R" and the rotatable structure 2, and is indicated by the arrows "Y" in figures 1 and 2. The arrows Y appear to be curved only in figure 2, because this embodiment provides for the trajectory followed by the cutting member 8, when it moves along the second direction, being circular around the rotation axis "R".

The combination of these movements, axial and circular around the axis, which are manageable independently of one another, enable the cutting member 8 to move along the entire lying surface 3 and thus follow any cutting trajectory.

Alternatively, the movement of the cutting member 8, along the direction "Y" can be replaced or combined with a similar rotation movement of the lying surface 3 itself.

The apparatus 1 further comprises expansion means 4 which enables deformation of the portion of leather more or less along the middle surface of the portion itself, and which in the illustrated embodiment is operationally interposed between the rotation means 5 and the rotatable structure 2.

In fact, in the preferred embodiment shown in the figures, the rotatable structure 2 comprises at least one sector 2a defining a part of the lying surface 3. The expansion means 4 is active on said at least one sector to translate the sector radially with respect to the rotation axis "R" of the rotatable structure 2, so as to increase or decrease the extent of the lying surface 3 and obtain the stretching or dilation of the portion of leather.

To increase this effect, the rotatable structure 2 comprises a plurality of sectors 2a, 2b and 2c defining respective adjacent parts of the lying surface 3. The expansion means 4 is active on these sectors 2a, 2b and 2c, to translate the sectors radially with respect to the rotation axis "R" of the rotatable structure 2, according to the arrows "A", "B", and "C".

To simplify the configuration of the apparatus 1, as is noted in figure 3, there is preferably provided at least one expansion means 4a, 4b or 4c respectively for each of the sectors 2a, 2b or 2c of the rotatable structure 2. This expansion means 4a, 4b and 4c determines the translation respectively of the sectors 2a, 2b and 2c of the rotatable structure 2, respectively according to the arrows "A", "B", and "C".

The expansion means 4 preferably comprises an actuator (4a,4b,4c) of electric and/or pneumatic type.

Preferably, the rotation means 5 of the rotatable structure 2 comprises a rotating support 5a suitable for dragging the rotatable structure 2 during rotation thereof around the rotation axis "R", and a motor 5b suitable for rotating the rotating support 5a.

Preferably, the expansion means 4 is operationally interposed between the rotation means 5 and the rotatable structure 2. In particular, the expansion means is operationally interposed between the rotating support 5a and the rotatable structure 2.

With reference to the movement means 10 of the cutting member 8, the movement means 10 comprises advantageously at least one slidable carriage 10a, with a suitable motor drive, by means of a main guide 10b, along at least said first direction parallel to the rotation axis "R" of the rotatable structure 2. Preferably, the movement means 10 comprises at least one curved guide 10c which is integral with the carriage 10a along the translation in the first direction.

The curved guide 10c extends transversely to the first direction and around the lying surface 3, as is noted in figures 1 and 2.

Preferably, the curved guide 10c follows at least one external profile of the lying surface 3, so as to be suitable for maintaining in contact the cutting member 8 with said at least one external profile, during translation of the cutting member 8, with a suitable motor drive, along the curved guide 10c. In this embodiment the external profile is circumferential, and the curved guide 10c is annular, such that the cutting member 8 can translate by travelling along a circular trajectory around the lying surface 3. Further, in this embodiment the cutting member 8 is integral with a cutting head 10d translating along the curved guide 10c.

Consequently, the axial sliding movement of the curved guide 10c on the carriage 10a defines the corresponding axial movement of the cutting member 8, whereas sliding of the cutting head 10d along the curved guide 10c defines the orbital movement of the cutting member 8.

From the point of view of the technical details that are implementable on the present invention, it must be pointed out that it is possible to interpose an intermediate support (known as the "blade-contrasting mat" or also "blade-saving mat") between the leather being processed and the cutting member 8: this intermediate support can thus consist of one or more "little mats"- typically arranged on different sectors of the lying surface 3 and makeable for example of felt but not only).

The function of the intermediate support consists of enabling the blade, during cutting, to traverse completely in depth the leather along the entire thickness thereof: consequently therefore the blade pierces for a few tenths or more of a millimetre also the intermediate support, without spoiling the underlying surface.

The intermediate support is makeable of transpirant material, so as to permit the action of sucking the leather and it is usually considered to be a consumable (especially as it is regularly replaced at preset operating intervals or when it has suffered a sufficiently high degree of deterioration through the effect of the blade).

Still from the point of view of the technical options that are associable with the present invention, it can be observed that the cutting head 10d can also carry several processing tools, for example tools for die-cutting and/or for making small holes, tools for making surface markings or marking in different manners the leather being processed and anything else. Depending on the type of additional tool mounted on the cutting head 10d, it is appropriately possible that the latter is positioned on a tangent line of the lying surface 3: for this purpose, additional orientation and drive means can be present that are suitable for permitting changes of the cutting head 10d along a supplementary movement axis, preferably in depth, and also suitable for performing operations of obtaining centring/coaxiality of work axes one or more of these additional tools with respect to the longitudinal/main extent axis of the lying surface 3.

The present invention achieves the proposed objects, overcoming the lamented drawbacks of the prior art.

The use of such a rotatable structure enables the spaces to be minimised and tensioning of the leather to be optimised, owing to the curved shape of the supporting surface that exploits the weight of the portion of leather. This improves the evidence of the defects, which can thus be easily detected.

Further, the structure of the members of the apparatus assigned to cutting the leather define an efficient and compact configuration and operate on portions of leather that are optimally subjected to traction/tensioning and thus with an optimal cutting action. The movement of the cutting member is moreover functionally optimal, being that it is able to follow automatically any trajectory around the lying surface.

## Claims

1. Apparatus (1) for the operations of analysing and cutting at least one portion of leather, comprising a structure (2) that defines at least one external lying surface (3), that is curved, for said at least one portion of leather and is suitable for supporting said at least one portion of leather, expansion means (4a, 4b, 4c), rotation means (5) and securing means (6) suitable for securing said at least one portion of leather to said rotatable structure (2), said structure being rotatable around a rotation axis (R) by said rotation means, one or more cutting members (8) and movement means (10) suitable for moving said one or more cutting members (8) on said lying surface (3), along at least one first direction (X) parallel to said rotation axis (R) of said rotatable structure (2) and a second direction (Y) transverse to said rotation axis (R) and means for the visual detection (7) of said at least one portion of leather, positioned in such a manner as to face said lying surface (3) during said rotation around said rotation axis (R),
wherein said securing means (6) comprises one or more grippers (6a, 6b) arranged preferably on said lying surface (3) and configured for grasping and retaining end strips of said at least one portion of leather, the securing means being preferably present on the surfaces that are perpendicular to the lying surface (3) for possible radial retaining of other end strips, **characterised in that** said structure (2) is expandable by said expansion means (4a, 4b, 4c), said expansion means (4a, 4b, 4c) being operationally interposed between said rotation means (5) and said rotatable structure (2),
wherein a first part (6a) of said securing means (6) and a second part (6b) of said securing means (6) are situated respectively on two opposite sides of said lying surface (3), and
wherein said rotatable structure (2) comprises at least one sector defining a part of said lying surface (3), and wherein said expansion means (4a, 4b, 4c) is active on said at least one sector to translate the sector radially or translate the sector rotationally to said rotation axis (R) of said rotatable structure (2), so as to increase or decrease the extent of said lying surface (3).

2. Apparatus (1) according to claim 1, wherein said rotatable structure (2) comprises at least one plurality of sectors (2a, 2b, 2c) defining respective adjacent parts of said lying surface (3), and wherein said expansion means (4a, 4b, 4c) is active on said sectors (2a, 2b, 2c) to translate radially or translate rotationally the sectors in relation to said rotation axis (R) of said rotatable structure (2), so as to increase or decrease the extent of said lying surface (3).

3. Apparatus (1) according to claim 1 or 2, wherein said lying surface (3) comprises at least part of a cylindrical surface, preferably said lying surface (3) being defined by a cylindrical surface.

4. Apparatus (1) according to one or more preceding claims, wherein said lying surface (3) is connected fluid-dynamically to sucking means (9) that is suitable for creating a vacuum on at least part of said lying surface (3).

5. Apparatus (1) according to claim 4 wherein said sucking means (9) is at least partially integrated into said rotatable structure (2).

6. Apparatus (1) according to one or more preceding claims, wherein said movement means (10) comprises at least one carriage (10a) that is slidable along at least said first direction (X) by means of a main guide (10b).

7. Apparatus (1) according to claim 6, wherein said movement means (10) comprises a curved guide (10c) which is integral with said carriage (10a).

8. Apparatus (1) according to claim 7, wherein said curved guide (10c) extends transversely to said first direction (X) and around said lying surface (3), said curved guide (10c) being preferably annular.

9. Apparatus (1) according to claim 7 or 8, wherein said curved guide (10c) follows at least one external profile of said lying surface (3) so as to be suitable for maintaining said cutting member (8) in contact with said at least one external profile during the translation of said cutting member (8) along said curved guide (10c) by a suitable motor drive.

10. Apparatus (1) according to one or more preceding claims, wherein said expansion means (4) comprises at least one actuator (4a, 4b, 4c) preferably of electric and/or pneumatic type.

11. Apparatus (1) according to one or more preceding claims, wherein said rotation means (5) comprises a rotating support (5a) suitable for dragging said rotatable structure (2) during rotation thereof around said rotation axis (R), and a motor (5b) suitable for rotating said rotating support (5a).

12. Apparatus (1) according to one or more preceding claims, wherein there is further an intermediate support that is interposable between the leather being processed and the cutting member (8), said intermediate support preferably consisting of one or more mats arranged on various sectors of the lying surface (3) and being makeable even more preferably of felt.

13. Apparatus (1) according to claim 12, wherein the intermediate support is makeable of transpirant and/or consumable material.

14. Apparatus (1) according to claims 12 or 13, comprising a cutting head (10d) suitable for carrying one or more additional tools, said processing instruments preferably comprising tools for die-cutting and/or for making small holes and/or tools for making surface markings and/or marking in different ways the leather fent being processed.

15. Apparatus (1) according to claim 14, wherein said one or more additional tools are positioned on a tangent line of the lying surface (3), the apparatus (1) further comprising additional orientation and drive means suitable for permitting movements of the cutting head (10d) along a supplementary movement axis, preferably in depth, and also suitable for performing operations of obtaining centring/coaxiality of work axes of said additional tools with respect to the longitudinal/main extent axis of the lying surface (3).

## Patentansprüche

1. Apparat (1) für Vorgänge zum Analysieren und Schneiden von mindestens einem Lederabschnitt, umfassend eine Struktur (2), die mindestens eine außenseitige liegende Oberfläche (3) definiert, die gekrümmt ist, für den mindestens einen Lederabschnitt, und die geeignet ist, um den mindestens einen Lederabschnitt zu stützen, Erweiterungsmittel (4a, 4b, 4c), Rotationsmittel (5) und Sicherungsmittel (6), die geeignet sind, um den mindestens einen Lederabschnitt an der drehbaren Struktur (2) zu sichern, wobei die Struktur durch die Rotationsmittel um eine Rotationsachse (R) drehbar ist, ein oder mehrere Schneidelemente (8) und Bewegungsmittel (10), die geeignet sind, um das eine oder die mehreren Schneidelemente (8) auf der liegenden Oberfläche (3) entlang mindestens einer ersten Richtung (X) zu bewegen, die parallel zur Rotationsachse (R) der drehbaren Struktur (2) angeordnet ist, und einer zweiten Richtung (Y), die quer zur Rotationsachse (R) angeordnet ist, und Mittel zur visuellen Erkennung (7) des mindestens einen Lederabschnitts, die so positioniert sind, dass sie der liegenden Oberfläche (3) während der Drehung rund um die Rotationsachse (R) zugewandt sind,
wobei die Sicherungsmittel (6) einen oder mehrere Greifer (6a, 6b) umfassen, die vorzugsweise auf der liegenden Oberfläche (3) angeordnet und ausgelegt sind, um Endstreifen des mindestens einen Lederabschnitts zu greifen und zu halten, wobei die Sicherungsmittel vorzugsweise auf den Oberflächen bereitgestellt sind, die senkrecht zur liegenden Oberfläche (3) angeordnet sind, für das mögliche radiale Halten anderer Endstreifen, **dadurch gekennzeichnet, dass** die Struktur (2) durch die Erweiterungsmittel (4a, 4b, 4c) erweiterbar ist, wobei die Erweiterungsmittel (4a, 4b, 4c) betriebswirksam zwischen den Rotationsmitteln (5) und der drehbaren Struktur (2) eingesetzt sind,
wobei ein erster Teil (6a) der Sicherungsmittel (6) und ein zweiter Teil (6b) der Sicherungsmittel (6) jeweils an zwei entgegengesetzten Seiten der liegenden Oberfläche (3) angeordnet sind, und
wobei die drehbare Struktur (2) mindestens einen Sektor umfasst, der einen Teil der liegenden Oberfläche (3) definiert, und wobei die Erweiterungsmittel (4a, 4b, 4c) auf den mindestens einen Sektor wirken, um den Sektor radial zu verschieben oder um den Sektor drehend zur Rotationsachse (R) der drehbaren Struktur (2) zu verschieben, sodass die Größe der liegenden Oberfläche (3) erhöht oder reduziert wird.

2. Apparat (1) nach Anspruch 1, wobei die drehbare Struktur (2) mindestens eine Vielzahl an Sektoren (2a, 2b, 2c) umfasst, die jeweilige angrenzende Teile der liegenden Oberfläche (3) definieren, und wobei die Erweiterungsmittel (4a, 4b, 4c) auf diese Sektoren wirken, um die Sektoren (2a, 2b, 2c) radial zu verschieben oder drehend in Bezug auf die Rotationsachse (R) der drehbaren Struktur (2) zu verschieben, sodass die Größe der liegenden Oberfläche (3) erhöht oder reduziert wird.

3. Apparat (1) nach Anspruch 1 oder 2, wobei die liegende Oberfläche (3) mindestens einen Teil einer zylindrischen Oberfläche umfasst und die liegende Oberfläche (3) vorzugsweise durch eine zylindrische Oberfläche definiert ist.

4. Apparat (1) nach einem oder mehreren der vorhergehenden Ansprüche, wobei die liegende Oberfläche (3) fluiddynamisch mit Saugmitteln (9) verbunden ist, die geeignet sind, um ein Vakuum auf mindestens einem Teil der liegenden Oberfläche (3) zu erzeugen.

5. Apparat (1) nach Anspruch 4, wobei die Saugmittel (9) mindestens teilweise in die drehbare Struktur (2) integriert sind.

6. Apparat (1) nach einem oder mehreren der vorhergehenden Ansprüche, wobei die Bewegungsmittel (10) mindestens einen Wagen (10a) umfassen, der mindestens entlang der ersten Richtung (X) mittels einer Hauptführung (10b) verfahrbar ist.

7. Apparat (1) nach Anspruch 6, wobei die Bewegungsmittel (10) eine gekrümmte Führung (10c) umfassen, die fest mit dem Wagen (10a) verbunden ist.

8. Apparat (1) nach Anspruch 7, wobei sich die gekrümmte Führung (10c) quer zur ersten Richtung (X) und rund um die liegende Oberfläche (3) erstreckt, wobei die gekrümmte Führung (10c) vorzugsweise ringförmig ist.

9. Apparat (1) nach Anspruch 7 oder 8, wobei die gekrümmte Führung (10c) mindestens einem außenseitigen Profil der liegenden Oberfläche (3) folgt, sodass sie geeignet ist, um das Schneidelement (8) in Kontakt mit dem mindestens einen außenseitigen Profil während der Verschiebung des Schneidelements (8) entlang der gekrümmten Führung (10c) durch einen geeigneten Motorantrieb zu halten.

10. Apparat (1) nach einem oder mehreren der vorhergehenden Ansprüche, wobei die Erweiterungsmittel (4) mindestens einen Steller (4a, 4b, 4c) umfassen, der vorzugsweise vom elektrischen und/oder pneumatischen Typ ist.

11. Apparat (1) nach einem oder mehreren der vorhergehenden Ansprüche, wobei die Rotationsmittel (5) eine rotierende Stütze (5a) umfassen, die geeignet ist, um die drehbare Struktur (2) während deren Drehung rund um die Rotationsachse (R) zu ziehen, und einen Motor (5b), der geeignet ist, um die rotierende Stütze (5a) zu drehen.

12. Apparat (1) nach einem oder mehreren der vorhergehenden Ansprüche, wobei zudem eine Zwischenstütze bereitgestellt ist, die zwischen dem bearbeiteten Leder und dem Schneidelement (8) eingesetzt werden kann, wobei diese Zwischenstütze vorzugsweise aus einer oder mehreren Matten auf verschiedenen Sektoren der liegenden Oberfläche (3) besteht und noch besser aus Filz gefertigt sein kann.

13. Apparat (1) nach Anspruch 12, wobei die Zwischenstütze aus einem durchlässigen Material und/oder Verbrauchsmaterial gefertigt sein kann.

14. Apparat (1) nach Anspruch 12 oder 13, umfassend einen Schneidkopf (10d), der geeignet ist, um ein oder mehrere zusätzliche Werkzeuge zu tragen, wobei diese Bearbeitungsinstrumente vorzugsweise Werkzeuge zum Stanzen und/oder Ausbilden kleiner Löcher und/oder Werkzeuge zum Herstellen von Oberflächenmarkierungen und/oder zum Markieren des bearbeiteten Lederstücks auf sonst eine Weise umfassen.

15. Apparat (1) nach Anspruch 14, wobei das eine oder die mehreren zusätzlichen Werkzeuge auf einer Berührungslinie der liegenden Oberfläche (3) positioniert sind, wobei der Apparat (1) zudem zusätzliche Ausrichtungs- und Antriebsmittel umfasst, die geeignet sind, um Bewegungen des Schneidkopfs (10d) entlang einer zusätzlichen Bewegungsachse, vorzugsweise in der Tiefe, zu erlauben, und auch geeignet sind, um Vorgänge zum Erhalten der Zentrierung/Gleichachsigkeit von Arbeitsachsen dieser zusätzlichen Werkzeuge im Vergleich zur Längs-/Hauptausdehnungsachse der liegenden Oberfläche (3) durchzuführen.

## Revendications

1. Appareil (1) pour les opérations d'analyse et de coupe d'au moins une portion de cuir, comprenant une structure (2) définissant au moins une surface d'appui extérieure (3), étant incurvée, pour ladite au moins une portion de cuir et étant adaptée pour supporter ladite au moins une portion de cuir, des moyens d'extension (4a, 4b, 4c), des moyens rotatifs (5) et des moyens de fixation (6) adaptés pour fixer ladite au moins une portion de cuir à ladite structure rotative (2), ladite structure pouvant pivoter autour d'un axe de rotation (R), grâce auxdits moyens rotatifs, un ou plusieurs organes de découpe (8) et des moyens de déplacement (10) adaptés pour déplacer lesdits un ou plusieurs des organes de découpe (8) sur ladite surface d'appui (3), le long au moins d'une première direction (X) parallèle au dit axe de rotation (R) de ladite structure rotative (2) et d'une seconde direction (Y) transversale au dit axe de rotation (R) et des moyens, destinés à la détection visuelle (7) de ladite au moins une portion de cuir, positionnés de manière à faire face à ladite surface d'appui (3) lors de ladite rotation autour dudit axe de rotation (R),
dans lequel lesdits moyens de fixation (6) comprennent un ou plusieurs préhenseurs (6a, 6b) disposés de préférence sur ladite surface d'appui (3) et configurés pour saisir et retenir des bandes terminales de ladite au moins une portions de cuir, les moyens de fixation étant de préférence présents sur les surfaces étant perpendiculaires à la surface d'appui (3) pour rendre possible la retenue radiale des autres bandes terminales, **caractérisé en ce que** ladite structure (2) peut être extensible grâce au moyens d'extension (4a, 4b, 4c), lesdits moyens d'extension (4a, 4b, 4c) étant fonctionnellement interposés entre lesdits moyens rotatifs (5) et ladite structure (2) pouvant pivoter, dans lequel une première partie (6a) desdits moyens de fixation (6) et une seconde partie (6b) desdits moyens de fixation (6) sont situées, respectivement, sur les deux côtés opposés de ladite surface d'appui (3), et
dans lequel ladite structure (2) pouvant pivoter comprend au moins un secteur définissant une partie de ladite surface d'appui (3), et dans lequel lesdits moyens d'extension (4a, 4b, 4c) sont actifs sur ledit au moins un secteur pour translater le secteur radialement ou le translater en rotation sur ledit axe de rotation (R) de ladite structure (2) pouvant pivoter, de manière à augmenter ou diminuer l'extension de ladite surface d'appui (3).

2. Appareil (1) selon la revendication 1, dans lequel ladite structure (2) pouvant pivoter comprend au moins une pluralité de secteurs (2a, 2b, 2c) définissant des parties adjacentes respectives de ladite surface d'appui (3), et dans lequel lesdits moyens d'extension (4a, 4b, 4c) sont actifs sur lesdits secteurs (2a, 2b, 2c) pour translater les secteurs radialement ou translater en rotation les secteurs par rapport au dit axe de rotation (R) de ladite structure (2) pouvant pivoter, de manière à augmenter ou diminuer l'extension de ladite surface d'appui (3).

3. Appareil (1) selon la revendication 1 ou 2, dans lequel ladite surface d'appui (3) comprend au moins une partie d'une surface cylindrique, de préférence ladite surface d'appui (3) étant définie par une surface cylindrique.

4. Appareil (1) selon l'une ou plusieurs des revendications précédentes, dans lequel ladite surface d'appui (3) est reliée de façon hydrodynamique à des moyens d'aspiration (9) étant adaptés pour créer un vide sur au moins une partie de ladite surface d'appui (3).

5. Appareil (1) selon la revendication 4, dans lequel lesdits moyens d'aspiration (9) sont au moins partiellement intégrés dans ladite structure (2) pouvant pivoter.

6. Appareil (1) selon l'une ou plusieurs des revendications précédentes, dans lequel lesdits moyens de déplacement (10) comprennent au moins un chariot (10a) pouvant coulisser le long d'au moins ladite première direction (X) au moyen d'un guide principal (10b).

7. Appareil (1) selon la revendication 6, dans lequel lesdits moyens de déplacement (10) comprennent un guide incurvé (10c) étant solidaire dudit chariot (10a).

8. Appareil (1) selon la revendication 7, dans lequel ledit guide incurvé (10c) se prolonge transversalement à ladite première direction (X) et autour de ladite surface d'appui (3), ledit guide incurvé (10c) étant de préférence annulaire.

9. Appareil (1) selon la revendication 7 ou 8, dans lequel ledit guide incurvé (10c) suit au moins un profil externe de ladite surface d'appui (3) de manière à pouvoir maintenir ledit organe de découpe (8) en contact avec ledit au moins profil externe lors de la translation dudit organe de découpe (8) le long dudit guide incurvé (10c) par un entraînement motorisé approprié.

10. Appareil (1) selon l'une ou plusieurs des revendications précédentes, dans lequel lesdits moyens d'extension (4) comprennent au moins un actionneur (4a, 4b, 4c) de préférence de type électrique et/ou pneumatique.

11. Appareil (1) selon l'une ou plusieurs des revendications précédentes, dans lequel lesdits moyens de rotation (5) comprennent un support de rotation (5a) pouvant tirer ladite structure (2) pouvant pivoter lors de sa rotation autour dudit axe de rotation (R), et un moteur (5b) pouvant faire tourner ledit support de rotation (5a).

12. Appareil (1) selon l'une ou plusieurs des revendications précédentes, dans lequel se trouve un support intermédiaire supplémentaire pouvant être interposé entre le cuir étant traité et l'organe de découpe (8), ledit support intermédiaire consistant de préférence en un ou plusieurs tapis disposés sur différents secteurs de surface d'appui (3) et étant constitués idéalement de feutre.

13. Appareil (1) selon revendication 12, dans lequel le support intermédiaire est constitué d'un matériau perméable et/ou consommable.

14. Appareil (1) selon revendication 12 ou 13, comprenant une tête de découpe (10d) pouvant transporter un ou plusieurs outils supplémentaires, lesdits instruments d'usinage comprenant de préférence des outils pour le découpage à l'emporte-pièce et/ou pour réaliser des petits trous et/ou des outils pour réaliser le marquage de surface et/ou marquer de différentes façons le cuir traité.

15. Appareil (1) selon revendication 14, dans lequel lesdits un ou plusieurs outils supplémentaires sont positionnés sur une ligne tangente de surface d'appui (3), l'appareil (1) comprenant également une orientation supplémentaire et des moyens d'entraînement pouvant permettre les déplacements des têtes de découpe (10d) le long d'un axe de mouvement supplémentaire, de préférence en profondeur, et pouvant également effectuer des opérations consistant à obtenir le centrage/la coaxialité des axes de travail desdits outils supplémentaires par rapport à l'axe d'extension longitudinal/principal de surface d'appui (3).
